# EUROPEAN PATENT SPECIFICATION

(11) **EP 3 162 394 B1**
(45) Date of publication and mention of the grant of the patent: **25.12.2019**
(21) Application number: 15811154.2
(22) Date of filing: 26.06.2015
(51) Int. Cl.: A61M 1/36, A61M 1/14, A61M 25/01

(54) **INTEGRATED DOUBLE CANNULA FOR ECMO**
INTEGRIERTE DOPPELKANÜLE FÜR ECMO
DOUBLE CANULE INTÉGRÉE POUR ECMO

(30) Priority: 26.06.2014 KR 20140004832 U
(43) Date of publication of application: 03.05.2017
(73) Proprietor: Samsung Life Public Welfare Foundation, Seoul 140-893 (KR)
(72) Inventor: CHO, Yang Hyun, Seoul 138-911 (KR)
(74) Representative: Patentanwälte Olbricht Buchhold Keulertz
(86) International application number: PCT/KR2015/006550
(87) International publication number: WO 2015/199486

(56) References cited:
- EP-A1- 1 440 663
- JP-A- 2005 278 905
- JP-A- 2005 278 905
- KR-A- 20130 088 455
- US-A- 5 009 636
- US-A- 5 009 636
- US-A1- 2005 085 761
- US-A1- 2005 085 761
- US-A1- 2008 161 739

## Description

### TECHNICAL FIELD

The present invention relates to an integrated double cannula for ECMO, and more particularly, to an integrated double cannula for ECMO configured to be inserted into a blood vessel, the integrated double cannula including a blood introduction part and a blood supply part integrally formed with a partition wall being therebetween, and a plurality of through-holes formed in a lateral portion of the integrated double cannula to increase the rate of blood flowing into an ECMO system.

### BACKGROUND ART

In general, extracorporeal membrane oxygenation (ECMO) refers to emergency cardiopulmonary bypass treatment for assisting bodily functions of patients such as the cardiopulmonary function of end-stage cardiac failure or respiratory failure patients.

ECMO is frequently used as emergency treatment for patients in cardiac arrest or failure due to coronary artery disease.

ECMO for providing temporary cardiopulmonary support to patients is generally of two types. One type is veno-arterial ECMO used to assist both the cardiac and pulmonary functions of patients, and the other type is veno-venous ECMO used to assist only the pulmonary function of patients. Particularly, in the veno-venous ECMO, cannulas connected to an end of an ECMO system are respectively inserted into a jugular vein of the neck and a femoral vein of a thigh. In most cases, blood drained through the cannulas connected to the femoral vein is circulated in the ECMO system and is then returned to the heart through the jugular vein. A cannula insertable into a jugular vein and enabling both the draining and returning of blood has been developed to substitute for the use of two cannulas at a time and thus to improve surgical procedures and patient convenience.

The above-mentioned cannula of the related art is made by connecting two cannulas side by side to have a diameter corresponding to the diameter of a jugular vein. However, the cannula of the related art is limited by the diameter of a jugular vein, that is, a blood vessel, and thus has a limited diameter, and the length of the cannula is also short. Therefore, the blood flow rate of the cannula is markedly limited.

### DETAILED DESCRIPTION OF THE INVENTION

### TECHNICAL PROBLEM

The patent document discloses a system for mixing a fluid into a bloodstream of a body, the system including: a drainage cannula adapted to be inserted through both a superior vena cava of a heart of the body and an inferior vena cava of the heart; the drainage cannula having a first opening to receive blood from a superior vena cava blood flow and a second opening to receive blood from an inferior vena cava blood flow; an infusion tube adapted to be inserted into a bloodstream downstream of both the superior vena cava and inferior vena cava; the drainage cannula and the infusion tube being coupled as a single cannula assembly with at least two lumens, one lumen being the drainage cannula and the other lumen being the infusion tube; a septum coupled between the lumens to fluidicly separate the lumens along at least a portion of a length of each lumen and adapted to flex between the two lumens to vary a cross-sectional flow area of at least one of the lumens; a pump coupled to the drainage cannula and the infusion tube; a mixing unit coupled to the pump and adapted to mix the fluid into the bloodstream of the body; and an atraumatic introducer coupled to the cannula assembly and sized larger than a cross-sectional dimension of at least one of the lumens and adapted to pass through such a lumen and cause the septum to flex toward another lumen to allow passage of the introducer.

However, the cannula is limited to the diameter of a jugular vein, that is, a blood vessel, and has a limited thickness and length. Thus, the blood flow rate of the cannula is markedly limited.

In addition, since an introduction part and an discharge port of the cannula through which blood is introduced and discharged are adjacent to each other, the cannula occupies a large space. Furthermore, since the cannula has only one side hole through which blood is discharged to an ECMO system, the amount of blood supplied to the EMC, that is, the flow rate of blood, is small, and thus blood is not smoothly oxygenated. As a result, a larger amount of blood has to be recirculated, and thus the efficiency of ECMO is low.

In addition, since the cannula of the related art is short, blood may not be smoothly drained and returned through the cannula inserted in a jugular vein of the neck of a patient if the patient moves his/her neck or face. Furthermore, the patient may be uncomfortable because of insertion of the cannula having a relatively large size.

US5009636 and US2005/0085761 A1 both disclose a dual-lumen catheter for use in extracorporeal oxygenation.

To solve the above-mentioned problems, the present invention provides an integrated double cannula for ECMO. The integrated double cannula is configured to be inserted into a femoral vein and includes two passages therein for introducing and supplying blood, wherein the two passages are defined by a partition wall, at least one through-hole is formed in a lateral portion of the cannula to increase the flow rate of blood flowing to an ECMO system, and even though a large amount of blood is introduced into the ECMO system, the large amount of blood is handled to increase the efficiency of surgical treatment.

### TECHNICAL SOLUTION

To achieve the above-mentioned objects, the present invention, which is defined in claim 1, provides an integrated double cannula configured to be inserted into a blood vessel for ECMO, the integrated double cannula including a cannula body in which a blood introduction part and a blood supply part are integrally formed inside a single pipe with a partition wall being therebetween, wherein an end of the cannula body is opened through an end hole, another end of the cannula body is opened through an outlet and an inlet arranged side by side and connected respectively to the blood introduction part and the blood supply part, and one or more side holes and one or more discharge holes connected respectively to the blood introduction part and the blood supply part are formed in a lateral portion of the cannula body. The partition wall is provided in the cannula body to separate the blood introduction part and the blood supply part, the partition wall comprises a flexible material and the side hole and the discharge hole are spaced apart from each other along a longitudinal direction of the cannula body.

The integrated double cannula further includes a ring-shaped metallic marker between the side holes and the discharge holes so that an end portion of the cannula body can be perceived in a radiographic image.

Preferably, a front end portion of the blood supply part has a slope inclined toward the outlet such that blood introduced through the end hole flows smoothly to an ECMO system.

Preferably, the flexible material of the partition wall is a film-shaped synthetic resin material.

Preferably, the integrated double cannula further includes a protective flange configured to be fitted into an insertion hole formed in skin to receive the cannula body.

### ADVANTAGEOUS EFFECTS OF THE INVENTION

The integrated double cannula for ECMO of the present invention has the following effects. First, owing to the one or more side holes, blood may be introduced into the ECMO system at a high rate, and even though a large amount of blood is introduced into the ECMO system, the blood may be handled, thereby increasing the efficiency of surgical treatment.

Secondly, owing to the one or more side holes, the flow rate of blood increases, and a blood recirculation phenomenon reduces markedly unlike in the related art, thereby increasing efficiency.

Thirdly, since the partition wall is formed of a film-shaped synthetic resin material, the shape of the partition wall may be freely varied to increase the space of the blood introduction part or the blood supply part, thereby increasing the flow rate of blood.

Fourthly, since the length of the cannula body is about 70 cm and greater than the length of conventional cannulas, even though the cannula body is somewhat moved inward or outward during surgical treatment, the surgical treatment may be safely performed without problems.

Fifthly, since the cannula body is configured to be inserted into a femoral vein, the integrated double cannula may be used for patients whose jugular veins are difficult to access, such as patients having surgical experience on their neck, jugular vein thrombosis, or a central vein catheter in their jugular vein, or patients in a very emergent situation such as a cardiopulmonary resuscitation situation.

Sixthly, during ECMO, the integrated double cannula of the present invention causes fewer complications such as heart injury than cannulas configured to be inserted into a cervical portion, and thus ECMO may be safely performed.

Seventhly, the protective flange may be fitted onto skin to receive the cannula while preventing the introduction of contaminants into a blood vessel.

### DESCRIPTION OF THE DRAWINGS

FIG. 1 is a view illustrating a structure of the related art.
FIG. 2 is a perspective view illustrating an integrated double cannula for ECMO according to the present invention.
FIG. 3 is a partially enlarged cross-sectional view illustrating a main portion of the integrated double cannula illustrated in FIG. 2.
FIG. 4 is a cross-sectional view illustrating a state in which the integrated double cannula of the present invention is used together with a protective flange.

### BEST MODE

Preferred embodiments of the present invention will now be described with reference to the accompanying drawings to help understanding of the present invention. The embodiments of the present invention may be variously modified, and thus it should not be construed that the scope of the present invention is limited to the embodiments described below in detail. The embodiments are provided to help those of ordinary skill in the art to fully understand the present invention. In the drawings, the shape of elements may be exaggerated for clear description. In addition, like reference numerals may denote like elements throughout the drawings. Furthermore, detailed descriptions related to well-known functions or configurations may be omitted in order not to unnecessarily obscure subject matters of the present invention.

Hereinafter, an integrated double cannula for ECMO will be described in detail according to preferred embodiments of the present invention with reference to the accompanying drawings.

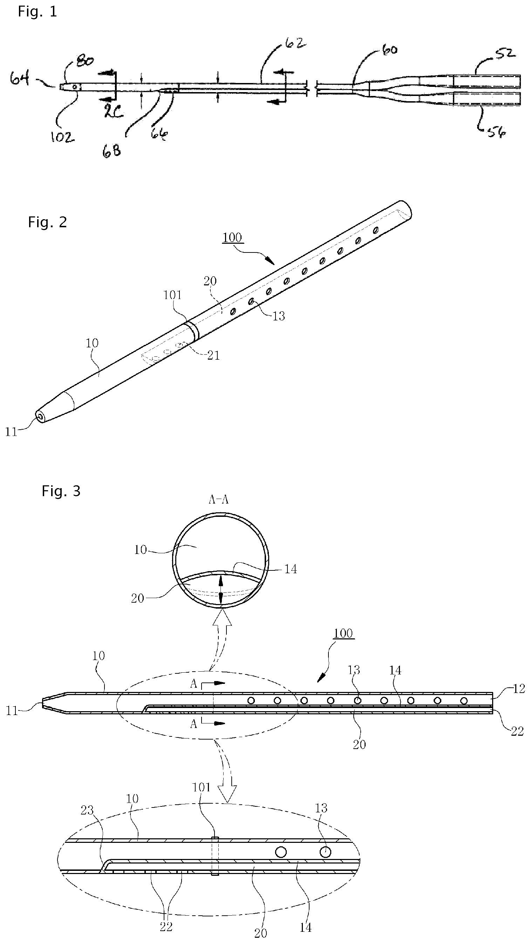

FIG. 2 is a perspective view illustrating the integrated double cannula for ECMO of the present invention, and FIG. 3 illustrates a cross section and an enlarged cross section of a main portion of the integrated double cannula illustrated in FIG. 2.

Referring to FIGS. 2 and 3, the integrated double cannula for ECMO of the present invention is shaped like a cylindrical pipe and includes a cannula body 100. The cannula body 100 is divided by an inner partition wall 14 into a blood introduction part 10 providing a blood path toward an ECMO system and a blood supply part 20 through which blood introduced into the ECMO system via the blood introduction part 10 is returned to a blood vessel after being oxygenated.

The blood introduction part 10 and the blood supply part 20 will now be described in detail. The blood introduction part 10 has a pipe shape, and an end hole 11 is formed in an end portion of the blood introduction part 10 to introduce blood. In addition, an outlet 12 is formed in another end portion of the blood introduction part 10 to discharge blood introduced through the end hole 11, and a plurality of side holes 13 are formed in a lateral portion of the blood introduction part 10.

The blood supply part 20 is separated from the blood introduction part 10 by the partition wall 14 formed on a side of the blood introduction part 10. A front portion of the blood supply part 20 is closed, and the blood supply part 20 includes a plurality of discharge holes 21 in a lateral portion thereof and an inlet 22 in a rear end portion thereof. Therefore, blood introduced into the blood introduction part 10 may be supplied back to a blood vessel after the blood passes through the ECMO system and the inlet 22. The partition wall 14 may be formed of a flexible material such as a synthetic resin material having a film shape. The synthetic resin material may be a plastic film such as a polyethylene film, an LDPE film, or an LLDPE film. In general, the partition wall 14 may be formed of a polyethylene film. Alternatively, the partition wall 14 may be formed of a recently developed biodegradable plastic film containing a biodegradable polymer. Therefore, as shown in the enlarged cross-section view of FIG. 3, since the partition wall 14 is formed of a flexible material such as a synthetic resin film, when blood is introduced into the blood introduction part 10, the partition wall 14 may be pushed toward a pipe inner wall, and thus an inner space of the blood introduction part 10 may be increased. Thus, the inflow rate of blood may be increased. In addition, when blood is supplied to the blood supply part 20, the partition wall 14 may be pushed inward and expanded, and thus an inner space of the blood supply part 20 may be increased. Therefore, the supply rate of blood may be increased.

In addition, a slope 23 inclined toward the outlet 12 is formed on a front end portion of the blood supply part 20, that is, a front end portion of the partition wall 14. Thus, blood introduced through the end hole 11 may be smoothly introduced into the ECMO system.

The cannula body 100 may be inserted into a femoral vein to supply oxygenated blood toward the tricuspid valve of the heart, and the blood introduction part 10 and the blood supply part 20 are integrally formed in a single pipe.

Therefore, the outlet 12 and the inlet 22 are arranged in parallel up and down the same site, and the blood supply part 20 is located on a side of the blood introduction part 10.

Preferably, the cannula body 100 may have a length of 65 cm to 75 cm, and most preferably 70 cm. The length from the outlet 12 and the inlet 22 to the last one of the side holes 13 is about 40 cm, and the length from the last side hole 13 to the end hole 11 is about 30 cm.

The cannula body 100 further includes a metallic marker 101 formed around a rear end region of the discharge holes 21 so that an end portion of the cannula body 100 may be easily perceived in a radiographic image. Therefore, when the cannula body 100 is inserted into a femoral vein, the insertion depth of the cannula body 100 may be easily checked using ultrasonic waves or X-rays.

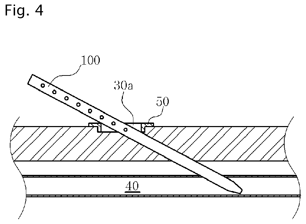

FIG. 4 is a cross-sectional view illustrating a state in which the integrated double cannula for ECMO is used together with a protective flange according to the present invention.

As illustrated in FIG. 4, preferably, the protective flange 50 is fitted in an insertion hole 30a formed in skin 30, and then the cannula body 100 is inserted into a blood vessel 40 through the insertion hole 30a of the skin 30. Thus, since a gap formed in the insertion hole 30a between the cannula body 100 and the skin 30 is blocked by the protective flange 50, foreign substances or contaminants may not be introduced into the blood vessel 40 through the skin 30, and thus the blood vessel 40 may not be contaminated.

### MODE OF THE INVENTION

Hereinafter, an explanation will be given of how the integrated double cannula having the above-described structure for ECMO is used according to the present invention.

First, the cannula body 100 is inserted into a femoral vein of a patient requiring ECMO.

Thereafter, as an ECMO system is operated, blood is introduced into the blood introduction part 10 through the end hole 11 and the side holes 13. Then, the inner space of the blood introduction part 10 is enlarged, and the blood is supplied to the ECMO system from the blood introduction part 10 through the outlet 12. In the ECMO system, the blood is oxygenated.

Thereafter, the oxygenated blood is introduced into the blood supply part 20 through the inlet 22, and then the inner space of the blood supply part 20 is enlarged. The oxygenated blood is supplied to the femoral vein from the blood supply part 20 through the discharge holes 21.

At this time, since the number of the side holes 13 is three or more, blood may be introduced into the ECMO system at a high rate. Although a large amount of blood is introduced into the ECMO system, the large amount of blood may be handled, and thus the efficiency of surgical treatment may be increased.

In addition, since the length of the cannula body 100 is about 70 cm, even though the cannula body 100 is somewhat moved inward or outward during surgical treatment, the surgical treatment may be safely performed without problems.

Furthermore, since the cannula body 100 is inserted into a femoral vein instead of inserting a conventional short cannula into a jugular vein, blood may be smoothly oxygenated without being largely affected by the movement of the neck and face of a patient.

While the integrated double cannula for ECMO has been described according to embodiments of the present invention, the embodiments should be construed in a descriptive sense only, and it will be apparent to those skilled in the art that various changes and other embodiments may be made therefrom. Thus, it will be understood that the present invention is not limited to the above detailed description. Therefore, the scope and spirit of the present invention should be defined by the claims of the present utility model application. In addition, it should be understood that the present invention covers all modifications, equivalents, and replacements within the spirit and scope of the present invention defined by the claims.

### INDUSTRIAL APPLICABILITY

According to the integrated double cannula for ECMO of the present invention, blood may be supplied to an ECMO system at a high rate, and even though a large amount of blood is introduced into the ECMO system, the large amount of blood may be handled, thereby increasing the efficiency of surgical treatment.

In addition, owing to the one or more side holes, the flow rate of blood increases, and a blood recirculation phenomenon reduces markedly. Thus, the integrated double cannula may be used to efficiently perform ECMO.

In addition, since the partition wall is formed of a film-shaped synthetic resin material, the shape of the partition wall may be freely varied to increase the space of the blood introduction part or the blood supply part. Thus, the integrated double cannula may have a high blood flow rate and thus may be usefully used for ECMO.

In addition, since the length of the cannula body is about 70 cm and greater than the length of conventional cannulas, even though the cannula body is somewhat moved inward or outward during surgical treatment, the surgical treatment may be performed without problems. Thus, the integrated double cannula of the present invention may be safely used for ECMO.

In addition, since the cannula body is configured to be inserted into a femoral vein, the integrated double cannula may be used for patients whose jugular veins are difficult to access, such as patients having surgical experience on their neck, jugular vein thrombosis, or a central vein catheter in their jugular vein, or for patients in a very emergent situation such as a cardiopulmonary resuscitation situation. Thus, the integrated double cannula may be usefully used for ECMO.

In addition, during ECMO, the integrated double cannula causes fewer complications such as heart injury than cannulas configured to be inserted into a cervical portion. Thus, the integrated double cannula may be safely used for ECMO.

In addition, the protective flange may be fitted onto skin to receive the integrated double cannula while preventing the introduction of contaminants into a blood vessel.

## Claims

1. A integrated double cannula configured to be inserted into a blood vessel for ECMO, the integrated double cannula comprising a cannula body (100) in which a blood introduction part (10) and a blood supply part (20) are integrally formed inside a single pipe with a partition wall (14) being therebetween, wherein an end of the cannula body (100) is opened through an end hole (11), another end of the cannula body (100) is opened through an outlet (12) and an inlet (22) arranged side by side and connected respectively to the blood introduction part (10) and the blood supply part (20), and one or more side holes (13) and one or more discharge holes (21) connected respectively to the blood introduction part (10) and the blood supply part (20) are formed in a lateral portion of the cannula body (100), the partition wall (14) is provided in the cannula body to separate the blood introduction part (10) and the blood supply part, the partition wall (14) comprises a flexible material, the side hole (13) and the discharge hole (21) are spaced apart from each other along a longitudinal direction of the cannula body (100), and **characterised in that** the integrated double cannula further comprises a ring-shaped metallic marker (101) between the side holes (13) and the discharge holes (21).

2. The integrated double cannula of claim 1, wherein an end portion of the cannula body (100) can be perceived in a radiographic image.

3. The integrated double cannula of claim 1, wherein a front end portion of the blood supply part (20) has a slope inclined toward the outlet such that blood introduced through the end hole (11) flows smoothly to an ECMO system.

4. The integrated double cannula of claim 1, wherein the flexible material of the partition wall (14) is a film-shaped synthetic resin material.

5. The integrated double cannula of claim 1, further comprising a protective flange (50) configured to be fitted into an insertion hole formed in skin to receive the cannula body (100).

## Patentansprüche

1. Integrierte Doppelkanüle, die konfiguriert ist, um in ein Blutgefäß für ECMO eingeführt zu werden, wobei die integrierte Doppelkanüle einen Kanülenkörper (100) umfasst, in dem ein Bluteinführungsteil (10) und ein Blutversorgungsteil (20) integral in einem einzigen Rohr mit einer Trennwand (14) dazwischen ausgebildet sind, wobei ein Ende des Kanülenkörpers (100) durch eine Endöffnung (11) geöffnet ist, ein anderes Ende des Kanülenkörpers (100) durch einen Auslass (12) und einen Einlass (22) geöffnet ist, die nebeneinander angeordnet und jeweils mit dem Bluteinführungsteil (10) und dem Blutversorgungsteil (20) verbunden sind, und eine oder mehrere Seitenöffnungen (13) und eine oder mehrere Ausgabeöffnungen (21), die jeweils mit dem Bluteinführungsteil (10) und dem Blutversorgungsteil (20) verbunden sind, in einem Seitenabschnitt des Kanülenkörpers (100) ausgebildet sind, die Trennwand (14) in dem Kanülenkörper vorgesehen ist, um den Bluteinführungsteil (10) und den Blutversorgungsteil zu trennen, die Trennwand (14) ein flexibles Material umfasst, die Seitenöffnung (13) und die Ausgabeöffnung (21) entlang einer Längsrichtung des Kanülenkörpers (100) voneinander beabstandet sind, und **dadurch gekennzeichnet, dass** die integrierte Doppelkanüle weiter einen ringförmigen metallischen Marker (101) zwischen den Seitenöffnungen (13) und den Ausgabeöffnungen (21) umfasst.

2. Integrierte Doppelkanüle nach Anspruch 1, wobei ein Endabschnitt des Kanülenkörpers (100) in einem Röntgenbild wahrnehmbar ist.

3. Integrierte Doppelkanüle nach Anspruch 1, wobei ein vorderer Endabschnitt des Blutversorgungsteils (20) eine Neigung zum Auslass hin aufweist, so dass das durch die Endöffnung (11) eingeleitete Blut reibungslos zu einem ECMO-System fließt.

4. Integrierte Doppelkanüle nach Anspruch 1, wobei das flexible Material der Trennwand (14) ein filmförmiges Kunstharzmaterial ist.

5. Integrierte Doppelkanüle nach Anspruch 1, weiter umfassend einen Schutzflansch (50), der konfiguriert ist, um in eine Einführungsöffnung in der Haut eingesetzt zu werden, um den Kanülenkörper (100) aufzunehmen.

## Revendications

1. Double canule intégrée configurée pour être insérée dans un vaisseau sanguin destinée à une ECMO, la double canule intégrée comprenant un corps de canule (100) dans lequel une partie d'introduction de sang (10) et une partie d'alimentation en sang (20) sont intégralement formées à l'intérieur d'un tuyau unique pourvu d'une paroi de séparation (14) se trouvant entre elles, dans laquelle une extrémité du corps de canule (100) est ouverte à travers un orifice d'extrémité (11), une autre extrémité du corps de canule (100) est ouverte à travers une sortie (12) et une entrée (22) disposées côté à côte et reliées respectivement à la partie d'introduction du sang (10) et la partie d'alimentation en sang (20), et un ou plusieurs orifices latéraux (13) et un ou plusieurs orifices d'évacuation (21) reliés respectivement à la partie d'introduction de sang (10) et la partie d'alimentation en sang (20) sont formés dans une partie latérale du corps de canule (100), la paroi de séparation (14) est prévue dans le corps de canule pour séparer la partie d'introduction de sang (10) de la partie d'alimentation en sang, la paroi de séparation (14) comprend un matériau souple, l'orifice latéral (13) et l'orifice d'évacuation (21) sont espacés l'un de l'autre selon une direction longitudinale du corps de canule (100), et **caractérisé en ce que** la double canule intégrée comprend en outre un marqueur métallique en forme de bague (101) entre les orifices latéraux (13) et les orifices d'évacuation (21).

2. Double canule intégrée selon la revendication 1, dans laquelle une partie d'extrémité du corps de canule (100) peut être observée dans une image radiographique.

3. Double canule intégrée selon la revendication 1, dans laquelle une partie d'extrémité avant de la partie d'alimentation en sang (20) présente une pente inclinée vers la sortie de sorte que le sang introduit par l'orifice d'extrémité (11) s'écoule doucement vers un dispositif d'ECMO.

4. Double canule intégrée selon la revendication 1, dans laquelle le matériau souple de la paroi de séparation (14) est un matériau de résine synthétique en forme de film.

5. Double canule intégrée selon la revendication 1, comprenant en outre un rebord protecteur (50) configuré pour être adapté dans un orifice d'insertion formé dans la peau pour recevoir le corps de canule (100).
